# EUROPEAN PATENT APPLICATION

(11) **EP 1 548 126 A1**
(43) Date of publication of application: **29.06.2005**
(21) Application number: 03293283.2
(22) Date of filing: 22.12.2003
(51) Int. Cl.: C12Q 1/68

(54) **Solid support for control nucleic acid, and application thereof to nucleic acid detection**

(71) Applicant: Bio-Rad Pasteur, 92430 Marnes-la-Coquette (FR)
(72) Inventor: Horvais, Alain, 95220 Herblay (FR); Biron, Marie-Philippe, 92600 Asnières sur Seine (FR)
(74) Representative: Paris, Fabienne

(57) **Abstract**

The present invention relates to solid supports for control nucleic acids. The present invention also relates to methods for the manufacture thereof, and methods of use thereof.

## Description

### Technical field

This invention relates to the field of nucleic acid detection. More precisely, this invention concerns solid supports for nucleic acid detection. These solid supports are specially adapted to nucleic acid control templates in an amplification reaction, such as PCR.

### Background art

Polymerase chain reaction (PCR) and PCR-based methods are known in the art (*Molecular Cloning: A Laboratory Manual,* Maniatis, Fritsch, and Sambrook, CSHL *Press*; *Molecular Biology of the Cell,* Alberts et al.).
These methods are very powerful tools for molecular biologists. PCR and PCR-based methods can be used for molecular cloning, DNA fragment preparation, complex sample analysis, in particular detection of given sequences.
A particularly important aspect of a PCR method is the use of a control. In most of the cases, controls allow for validation of the PCR conditions, as detailed below.
PCR is generally performed in parallel in several separate tubes. Each of these tubes typically contains a sample template, together with a certain amount of a so-called PCR reagent mix. Said PCR reagent mix usually contains most of the required reagents for the PCR reaction, except for the templates to be amplified. A positive control generally involves the use of a known template, which is known and expected to yield an amplification product in the desired PCR conditions, if all desired conditions are actually fulfilled. Through the use of a positive control, it is thus usually possible to assess whether the PCR conditions were suitable in a given experiment, e.g. if the salt conditions, including magnesium concentration, were suitable for the PCR reaction to occur, or if the polymerase was present in suitable amounts. In other words, the use of a positive control typically serves as a validation of the PCR conditions: if the positive control does not yield any amplification product, then it usually disqualifies the other results in the parallel PCR tubes, because the other negative results cannot be validly interpreted.
As it is known from the skilled person, positive controls are also generally essential in real-time PCR experiments, and in quantitative PCR experiments.
However, as the other templates in the experiment, the template used for the positive control is typically provided in solution under liquid form. In addition, it is quite frequent, that the volume to be dispensed for such a template solution is very small, in the range of a few microliters. Hence, it is sometimes difficult to assess, based on volume measurements, whether or not the desired template has been introduced into the reaction tube. This also applies even before the PCR cycles.
However, in case there is no amplification in the tube for the positive control, one generally needs to ensure that the positive template control had actually been added. This hypothesis often needs to be checked, in order to identify which parameter(s) have to be modified in order to obtain an amplification product.

Hence, there is a need for devices and methods, which allow for a quick, easy, absolute, unambiguous and reliable check for the presence of a control template in a given sample.
In addition, there is a need not only for qualitative control solid supports, but also for quantitative control solid supports, especially for use in complex media.

Solid supports for nucleic acid templates are known in the art, but none of them have been designed with the purpose of addressing the problem which is now solved by the present invention. As a matter of fact, such prior art supports cannot address it: even in the light of the present invention, such prior art supports do not prove to be satisfactory with respect to their ability to reproducibly release adsorbed nucleic acid templates. In this view, they absolutely cannot assume the function of supports for quantitative control templates, especially in complex fluids such as urine or plasma. As a contrary, such prior art supports are optimized for DNA storage.
For example, US 5 939 259 and US 6 168 922 describe solid supports for nucleic acid storage, from which said nucleic acid can be released. However, these supports comprise a chaotropic salt.
US 5 807 727 describes a solid medium for DNA storage, but this prior art medium necessarily comprises a compound having a protecting effect against degradation, typically a detergent.

The inventors therefore have designed and produced solid supports which can assume both functions of qualitative and quantitative controls. As a remarkable feature, to assume their functions, the solid supports according to the invention do not require the presence of chaotropic salt, nor of any other compound like a detergent.
Very notably, the supports according to the invention allow for control templates to be desorbed in a reproducible fashion when contacted with a liquid. Remarkably, this feature is conserved even with complex fluids and mediums (especially such as biological samples from a human or an animal, e.g. urine, blood, plasma, etc), whereas it is observed that prior art supports fail to meet such reproducibility levels, which would be required for them to be used as membranar control supports, for instance in a PCR amplification.
In addition, due to their structure, material and dimensions, the solid supports according to the present invention offer the advantage of being extremely easy to handle. In particular, because they are rigid enough, and essentially devoid of electrostatic effects, they can be easily and quickly distributed using a manual, semi-automatic or automatic dispenser, which makes them particularly useful for many molecular biology experiments both on the laboratory and on a larger scale.

### Description of the invention

This is an object of the present invention to provide with a method to determine the presence or the absence of at least one target nucleic acid by reference to at least one control nucleic acid.

According to the present invention, there is provided a method to determine the presence or the absence of at least one target nucleic acid by reference to at least one control nucleic acid, which comprises processing said target nucleic acid so as to allow its detection, submitting said control nucleic acid to comparable processing conditions, and validating or invalidating the detection result obtained for said target nucleic acid by comparing it to the detection result obtained for said control nucleic acid,
wherein said control nucleic acid is provided by a solid support onto which it is adsorbed, and from which a definite amount thereof is to be desorbed, whereby there is provided an essentially quantitatively reproducible and controlled amount of said control nucleic acid for submission to said comparable processing conditions.

By detection of an item, we hereby understand any process which essentially allows for the determination of the presence, when applicable, and/or the determination of amount, when applicable, of said item.
By nucleic acid, we hereby understand any nucleic acid: it can be synthetic or not, recombinant or naturally occurring, linear or circular. This includes DNA and RNA.

The nucleic acid can be either single stranded or double stranded or even triple stranded. It can stem from various biological sources, such as micro organisms (bacteria, yeasts, and the like), or higher organisms, like mammal cells. Said nucleic acid can also be of viral nature, e.g. retroviral nature, like HIV's. The nucleic acid can also comprise total DNA, total RNA, genomic DNA, mitochondrial DNA, plasmidic DNA, BAC DNA, and mixtures thereof. Moreover, the nucleic acid can assume various states of purity.
By target nucleic acid, we hereby understand any nucleic acid, whose presence, when applicable, and/or amount, when applicable, is/are to be detected.

In one embodiment, there is provided a method for the detection of at least one target nucleic acid, which method comprises the steps of:
- providing with said solid support comprising said at least one control nucleic acid adsorbed thereon, and contacting said solid support with a liquid medium, so as to allow a definite amount of said control nucleic acid to be desorbed from said solid support into said liquid medium, substantially without affecting the primary sequence of said control nucleic acid;
- optionally, further processing the resulting liquid medium containing said control nucleic acid;
- submitting said control nucleic acid which is contained in or originating from said resulting, optionally further processed, liquid medium, to comparable processing conditions, so that it constitutes a processing control by reference to the processing of said target nucleic acid;
- determining whether said target nucleic acid is detected or not, and determining whether said control nucleic acid is detected or not;
- comparing the target nucleic acid detection result to the one of said control nucleic acid, so as to validate or invalidate the target nucleic acid detection result.

By solid support, we hereby understand any material assuming a solid state in the temperature range from 0°C to 110°C. In particular, solid supports include those supports, whose thickness and rigidity allow for easy handling, especially in a temperature range of 0-50°C, preferably 15-30°C, more preferably around room temperature (25°C).
The wording 'control' is clear to those skilled in the art. In particular, it refers to any element in a a process, which contributes to the validation or non-validation of the general conditions of said process.
The wording 'liquid medium' is known to those skilled in the art. In particular, this is construed as to encompass any medium which can assume a liquid state at a temperature in the range 4-70°C, more particularly from room temperature (25°C) to human body temperature (37°C). Such liquid media include various solutions known to those skilled in the art, for example aqueous solutions, buffers, water, injection-grade water, etc. Liquid media also include some fluids such as bodily fluids, urine, plasma, blood, cerebrospinal fluid. Liquid media thus also include fluid biological media. Said liquid medium can be a sample, e.g. a biological sample.

By primary sequence, we hereby understand the following. In the case of nucleic acids, the primary sequence includes the sequence of bases along one nucleic acid molecule strand.

According to the invention, said processing step may comprise at least one of any processing step commonly known in the art. In particular, said processing step may comprise at least one of any processing step for detection. As understood herein, detection comprises any detection process or method for assessing the presence or absence or amount of nucleic acids (qualitative and/or quantitative detection). In particular, detection includes any process or method applied to samples susceptible of containing at least one nucleic acid, aiming at the determination of the presence or absence or amount of said nucleic acid in said sample. Such detection methods and processes are known in the art. Examples thereof include, but are not limited to, amplification, including PCR amplification, electrophoresis, including gel electrophoresis such as polyacrylamide or agarose gel electrophoresis, and capillary electrophoresis, Southern analysis, northern analysis, hybridization including probe-mediated hybridization, blot hybridization, and combinations thereof. Such techniques are known to those skilled in the art (Molecular Cloning: A Laboratory Manual, Maniatis, Fritsch, and Sambrook, CSHL Press).

By amplification, we hereby understand any sort of process involving an amplification step as known in the art. This can be e.g. a PCR amplification, or any other amplification, PCR-based or not.

By PCR or PCR reaction, we hereby understand any PCR-based reaction. This includes standard PCR, quantitative and semi-quantitative PCR, multiplex PCR, e.g. duplex or triplex PCR, as well as RT-PCR (reverse transcriptase PCR), real time PCR and the like.

Where detection includes amplification, such as PCR amplification, said control nucleic acid can thus serve as a control nucleic acid template (or, as equivalently used herein, control template), and said target nucleic acid is thus possibly a target nucleic acid template (or, as equivalently used herein, target template).
The wording 'template' or 'template in an amplification reaction' or 'control template' is clear to those skilled in the art. In particular, this is understood to include any nucleic acid or molecule which is to be, at least partly, amplified in an amplification reaction. Said template can have various lengths, typically 0.050-100 kb, preferably 0.050-10 kb, more preferably 0.1-5.0 kb. Said template can lead to the generation of an amplicon in the course of an amplification reaction.

By hybridization, we hereby understand any method involving detection through hybridization under given stringency conditions. As known in the art, hybridization generally involves the use of a probe for the detection of a target nucleic acid.

Advantageously according to the invention, there is thus provided a method for the detection of target nucleic acids by reference to control nucleic acids. Said method finds applications in many respects, and especially for detection of nucleic acids within samples, such as biological samples.
By biological sample, it is hereby understood any biological material. Examples of biological samples include samples or fluids comprising serum, blood, urine, spinal fluid, feces, plasma, buccal cells, tissue culture samples, cell culture samples, plant samples, microbiological cultures, bacterial cultures, and the like. Other examples include nucleic acid solutions, in any solvent known in the art. Example of such solvents include water, distilled water, injection-grade water, nuclease-free water, TE, etc. (*Molecular Cloning: A Laboratory Manual,* Maniatis, Fritsch, and Sambrook, CSHL Press).

Advantageously according to said method, there is thus provided a mean for an easy, quick, absolute, unambiguous and reliable check for the presence of a control, and thus allows for easier validation or invalidation of experimental data.
Said support having said control nucleic acid adsorbed thereon can directly be placed in a laboratory tube. Thus, presence of said control template can be quickly, easily, absolutely, unambiguously and reliably assessed by visual check: The presence (or absence) of said control nucleic acid in said tube is advantageously indicated by the presence (or absence) of said support in said tube - and conversely. When adding a liquid medium to said tube, any control nucleic acid adsorbed onto the support is essentially quantitatively reproducibly desorbed, in a controlled fashion. Thus, by simply adding e.g. water or buffer, said control nucleic acid comes into solution by desorption. Presence of said template is thus confirmed by the presence of said solid support. It is then possible to remove and discard said solid support, or simply leave it in the liquid medium. Moreover, such a support might be used as a control during a nucleic acid recovery and/or purification procedure. According to the invention, said liquid medium allows for said control nucleic acid to be essentially quantitatively reproducibly desorbed, in a controlled fashion, when contacted with a liquid medium. This occurs advantageously without substantial alteration in the primary sequence of said template. Thus said support having a control nucleic acid adsorbed thereon, might simply be added to a sample to be analyzed. After the control nucleic acid has been desorbed, it is possible to carry out a processing step, e.g. nucleic acid purification and/or extraction (recovery) on the resulting liquid medium. Presence of said control nucleic acid after processing will allow for validation of the processing step. Because its primary sequence is substantially unaltered, said control template can then advantageously be further processed for detection, for instance in an amplification reaction like PCR. Because the actual nature of the processing for detection is not per se limited, said method according to the present invention is advantageously useful for most, if not all, methods for nucleic acid detection where a control nucleic acid is involved.

A particularly preferred embodiment of the method according to the present invention relates to the field of amplification.

In particular, said processing for detection can comprise at least one amplification step, preferably a PCR amplification step.
The method according to the invention is particularly advantageous in the field of PCR-mediated detection (analytical PCR), where the use of control nucleic acids is a crucial issue. This especially applies to quantitative PCR, where not only qualitative, but also quantitative controls are required.

Advantageously according to the invention, said support allows for said control nucleic acid to be desorbed reproducibly and in a controlled fashion, when placed into contact with a liquid medium. This is particularly important in the case of semi-quantitative or quantitative PCR: the starting amount of control nucleic acid is advantageously accurately controlled. Thus, there is no error due to inexact pipetting into the tube. According to the invention, the control nucleic acids can be reproducibly and quantitatively released from the support material. Presence of the template is also easily checked before the further processing for amplification is started (visual check).
The skilled person would know how to carry out such a PCR amplification step, and in particular, how to choose suitable amplification reaction conditions. The skilled person would also know how to choose suitable controls, and interpret the results therefrom (validation or invalidation of said detection resulrs).
By PCR amplification reaction conditions, we hereby mean all the conditions under which a PCR reaction is usually performed. This includes the common salt (ionic force, magnesium concentration range), temperature, stringency, enzymatic and cycling, conditions.
According to the invention, said method then comprises the step of providing with the required reagents for said amplification. Advantageously, at least some of said required reagents are provided under the form of a suitable PCR reagent mix. The skilled person is aware of the components present in such a PCR reagent mix. (Molecular Cloning: A Laboratory Manual, Maniatis, Fritsch, and Sambrook, CSHL Press). Examples of components of a PCR reagent mix include distilled water or sterile water or DNAse free water or RNase free water, magnesium ions, at least one polymerase like the Taq, the Vent, various salts like magnesium, oligonucleotides used as primers for the PCR reaction including polyT, dyes such as SyberGreen™, internal probes like Taqman probes or molecular beacon probes (using dyes known in the art like FAM, HEX, JOE,TET, VIC, Cy3, Cy5, ROX, TAMRA, LC 640, LC 705 and the like, and quenchers known in the art like Dabcyl, Black Hole quencher, Eclipse Dark quencher and the like), deoxynucleotides, nucleotides, ribonucleotides, dNTPs, dATP, dTTP, dCTP, dGTP, and derivatives thereof, e.g. fluorophore-tagged. The skilled person would know how to choose suitable primers and probes, together with the suitable final concentrations for said components (Molecular Cloning: A Laboratory Manual, Maniatis, Fritsch, and Sambrook, CSHL Press).
In a preferred embodiment according to the invention, said PCR reagent mix comprises all the reactants required for the PCR reaction, except for the template. For example, it is possible to use a PCR reagent master-mix. The skilled person is aware of how to prepare such a reagent master-mix. Advantageously, said reagent master-mix comprises common components required for the PCR reaction, in a volume sufficient to be dispensed into all desired reaction tubes. In this way, reaction conditions can be as comparable as possible, because said common components originate from the same PCR reagent master-mix.

In said method according to the invention, said solid support can be provided under any shape or any form. In a preferred and particularly advantageous embodiment, said solid support comprises at least one membrane. Preferred materials for said membrane include materials selected from the group constituted by cellulose, cellulose-derived materials including chemically-treated celluloses, glass fibers, nylons, polyethersulfones, polypropylene, woven porous polymers, non-woven porous polymers, PTFE, porous glasses, and PVDF. Amongst those materials, particularly preferred materials include those selected from the group constituted by cellulose, cellulose-derived materials including chemically-treated celluloses, glass fibers, nylons, polyethersulfones, and polypropylene. The most preferred materials are selected from the group constituted by cellulose, cellulose-derived materials and nylons.
In said method according to the invention, there is little limitation about the shape and dimensions of said membrane. In one embodiment, said membrane has a thickness in the range 50-3000 microns, preferably 100-1500 microns, more preferably 150-1000 microns. In another embodiment, said membrane has the shape of a disk, a square, a rectangle or a strip.

In said method according to the invention, said solid support advantageously further comprises at least one carrier agent adsorbed thereon. Said carrier agent advantageously contributes to providing with an essentially quantitatively reproducible and controlled amount of said control nucleic acid for submission to said comparable processing conditions. Said carrier agent is an agent having a carrier effect on nucleic acids, as can be judged by those skilled in the art. It can be any carrier agent, as long as it does not interfere in the detection processing of said control nucleic acid and/or in the detection of said target nucleic acid. In one embodiment, said carrier agent is selected from the group constituted by:
- nucleic acids unrelated or heterologous to said control nucleic acid, so as to advantageously substantially not interfere in the detection processing of said control nucleic acid;
- proteins.
In said method according to the invention, said carrier agent can preferably be a nucleic acid unrelated or heterologous to said control nucleic acid and also unrelated or heterologous to said target nucleic acid, so as to substantially not interfere in the detection processing of said control and said target nucleic acids, especially in the case of amplification such as PCR. Examples for said carrier agent are selected from the group constituted by polyA, polyG, polyC, polyT, polydA, polydG, polydC, polydT, polydU, homopolydeoxyribonucleotides, homopolyribonucleotides , block-polymers of deoxyribonucleotides, block-polymers of ribonucleotides, block-polymers of deoxyribonucleotides and ribonucleotides, and mixtures thereof. In another embodiment, said carrier agent is selected from the group constituted by:
- fish DNA, such as herring DNA, especially herring sperm DNA, and salmon DNA, especially salmon sperm DNA, and mixtures thereof;
- albumins, especially bovine serum albumin (BSA). In a preferred embodiment according to the invention, said carrier agent is selected from the group constituted by:
- homopolydeoxyribonucleotides, block-polymers of deoxyribonucleotides, and mixtures thereof;
- albumins, especially bovine serum albumin (BSA). Advantageously in the methods according to the invention, such carrier agents have proven to be generally of high performance for most processing steps for detection, in particular in the framework of PCR, quantitative PCR and/or real-time PCR.
Other putative carrier agent candidates were tested by the Applicant, and were found to be unsatisfactory in their capacity to release said control nucleic acid from said solid support in a reproducible fashion. For example, the Applicant tested sugars as carrier agents, and in particular saccharose (Merck, ref. 1.07687.1000). However, the use of saccharose resulted in poor reproducibility in the release of the control nucleic acid from the solid support.

The present invention thus provides with a method involving control nucleic acids. Said control nucleic acid can be of various types, as is known to those skilled in the art. Advantageously according to the invention, said control nucleic acid can be selected from the group constituted by positive controls, negative controls, internal controls, external controls, qualitative controls, semi-quantitative controls, quantitative controls, real-time amplification controls, and combinations thereof. Said method is thus useful in many cases, if not all, where a control nucleic acid comes into play.

Said method according to the invention may also comprise further steps. In particular, it is possible for said nucleic acid processing for detection to comprise multiple and/or further steps.
In one embodiment, said processing step comprises at least one nucleic acid extraction and/or purification step, prior to said nucleic acid detection step.
In another embodiment, said processing step comprises a detection step involving at least one hybridization step.
In yet another embodiment, said processing comprises a detection step involving at least one PCR or RT-PCR, especially real-time and quantitative PCR or RT-PCR.
Preferably according to the invention, said target nucleic acid detection is a quantitative detection, preferably a real-time quantitative detection.
Also preferably according to the present invention, said control nucleic acid detection is a quantitative detection, preferably a real-time quantitative detection.
All such steps are known to those skilled in the art (*Molecular Cloning: A Laboratory Manual,* Maniatis, Fritsch, and Sambrook, CSHL Press).

Preferably, the method for detection according to the present invention is to be carried out under nuclease-free conditions.

In another aspect, it is a further object of the present invention to provide with solid supports for nucleic acids.

In particular, the invention provides with a solid support for at least one control nucleic acid, said solid support being specifically adapted for carrying out a method for the determination of presence or absence of at least one target nucleic acid by reference to at least one control nucleic acid according to the invention, as described above.
According to the invention, there is provided a solid support being specifically adapted for carrying out a method for the determination of presence or absence of at least one target nucleic acid by reference to at least one control nucleic acid according to the invention, wherein said solid support comprises:
- at least one absorbent support made of a material whose composition and structure allow for non-covalent adsorption of said control nucleic acid onto said solid support, and which is or has been heat-treated and/or chemically-treated so as to be essentially devoid of any enzymatic activity;
- at least one carrier agent adsorbed thereon, which facilitates the adsorption of said control nucleic acid onto said solid support and/or facilitates the desorption of said control nucleic acid from said solid support and/or promoting the stability of said control nucleic acid on said solid support, especially in the course of storage, substantially without affecting the primary sequence of said control nucleic acid.

Said support material can be either non-hydrophobic or hydrophobic. Any size and shape are possible for the solid support according to the present invention.
By absorbent, we understand any material having an affinity for aqueous solutions which is high enough, as to be able to absorb essentially instantaneously a solution without any specific chemical pre-treatment.
By structure, we understand any structural feature characterizing the material. This includes macro structure (e.g. presence of layers, sub-parts, composite structures,...) as well as micro structure (e.g. crosslinking level, pore density, average pore size, ...)
By non-covalent adsorption, we hereby understand any kind of chemical bonding, which is not strictly covalent. This includes ionic bonding, hydrogen bonding, electrostatic bonding, e.g. through van der Waals forces, dipole interactions ...
By enzymatic activity, we hereby understand any catalytic activity resulting from an enzyme. This includes protease activity, nuclease activity, in particular endo- and exonuclease activity, as well as DNAse (DNAse I, restriction enzymes, etc.) and RNase activity (e.g. RNase I). Preferably, said enzymatic activity comprises nuclease activity, including DNAse and/or RNase activity.
By carrier agent, we hereby understand any agent having a carrier effect on nucleic acids, as can be judged by those skilled in the art. It can presently be any agent which facilitates the adsorption of said control nucleic acid onto said solid support and/or facilitates the desorption of said control nucleic acid from said solid support and/or promoting the stability of said control nucleic acid on said solid support, especially in the course of storage, substantially without affecting the primary sequence of said control nucleic acid. In one embodiment, said carrier agent is selected from the group constituted by:
- nucleic acids unrelated or heterologous to said control nucleic acid and/or to said target nucleic acid, so as to generally not interfere in the detection method;
- carrier agents of proteic nature.
The skilled person would know how to choose such a carrier agent. Said carrier would advantageously be unrelated to said target nucleic acid, or to said control nucleic acid, or to both.
In a preferred embodiment, said carrier agent is a nucleic acid unrelated to any naturally occurring human nucleic acid. Thus, said carrier agent would advantageously essentially not interfere in the detection of human nucleic acids (as control and/or target).
In another preferred embodiment, said carrier agent is a nucleic acid unrelated to any nucleic acid originating from nucleic acid originating from any naturally occurring agent being pathogen to a mammalian, especially to human. Thus, said carrier agent would advantageously generally not interfere in the detection of nucleic acids originating from pathogens and micro-organisms such as yeasts, fungi, bacteria, viruses, and the like (as control and/or target).
In a preferred embodiment, said carrier agent is selected from the group constituted by:
- polyA, polyG, polyC, polyT, polydA, polydG, polydC, polydT, polydU, homopolydeoxyribonucleotides, homopolyribonucleotides , block-polymers of deoxyribonucleotides, block-polymers of ribonucleotides, block-polymers of deoxyribonucleotides and ribonucleotides, and mixtures thereof;
- fish DNA;
- proteins, such as albumin.
Preferably, said carrier agent is selected from the group constituted by:
- homopolydeoxyribonucleotides such as polydA, block-polymers of deoxyribonucleotides, and mixtures thereof;
- herring DNA, especially herring sperm DNA, and salmon DNA, especially salmon sperm DNA, and mixtures thereof;
- albumins, such as bovine serum albumin (BSA).
In a most preferred embodiment, said carrier agent is selected from the group constituted by:
- homopolydeoxyribonucleotides such as polydA, block-polymers of deoxyribonucleotides, and mixtures thereof;
- albumins, such as bovine serum albumin (BSA).
Other putative carrier agent candidates were tested by the Applicant, and were found to be unsatisfactory in their capacity to release said control nucleic acid from said solid support in a reproducible fashion. For example, the Applicant tested sugars as carrier agents, and in particular saccharose (Merck, ref. 1.07687.1000). However, the use of saccharose resulted in poor reproducibility in the release of the control nucleic acid from the solid support.

The length (in kilobases, kb) of said polyA, polyG, polyC, polyT, polydA, polydG, polydC, polydT, polydU, homopolydeoxyribonucleotides, homopolyribonucleotides , block-polymers of deoxyribonucleotides, block-polymers of ribonucleotides, block-polymers of deoxyribonucleotides and ribonucleotides is not critical within the invention.
The amount of carrier agent on said solid support according to the invention is not limited, as long as it is present in an amount sufficient to contribute to an essentially quantitatively reproducible and controlled desorption of said control nucleic acid. In one embodiment, said carrier agent comprises 0.1-50 µg of nucleic acids, preferably 1-10 µg, more preferably 5-6 µg, even more preferably 4-8 µg for a 6mm × 6mm square membrane solid support. The skilled person would know how to adapt the amount of said nucleic acid carrier agent as a function of the size and dimensions of said solid support.
In another embodiment, said carrier agent comprises 2-100 µg of BSA, preferably 5-50 µg, more preferably 10-30 µg, even more preferably 15-20 µg for a 6mm × 6mm square membrane solid support. Again, the skilled person would know how to adapt the amount of protein as a function of the size and dimensions of said solid support.

Advantageously according to the invention, said solid support is particularly easy to handle, rather rigid, and is not subject to electrostatic effects. This makes it particularly useful for general laboratory manipulations.

In one embodiment, said solid support according to the invention further comprises a control nucleic acid adsorbed thereon. Said control nucleic acid can be any type of control nucleic acid known in the art. In a preferred embodiment, said control nucleic acid is selected from the group constituted by positive controls, negative controls, internal controls, external controls, qualitative controls, semi-quantitative controls, quantitative controls, real-time amplification controls, and combinations thereof. Depending upon the target nucleic acid to be detected, the skilled person would know how to choose such controls, and their relevancy in the detection process. For example, in the case of a negative control, it is usually useful to choose a control nucleic acid which would not be detected through the detection of said target nucleic acid. Similarly, in the case of a positive control, the skilled person would choose a control nucleic acid being prone to generate a signal in the detection. Said control nucleic acid adsorbed onto said solid support can be present in various amounts. Depending upon the needs, and the nature of the detection, the skilled person would know which amounts are suitable or desirable. For example, said control nucleic acid is adsorbed in an amount in the range 10-10⁸ copies, preferably 10²-10⁵ copies. It can also be envisaged that said control nucleic acid is adsorbed in an amount in the range 10-1000 copies, preferably 20-500 copies, more preferably 50-100 copies. Advantageously according to the invention, the amount of control nucleic acid adsorbed onto said solid support is known and determined. This possibility is particularly adapted for quantitative detection, e.g. quantitative PCR.
Very advantageously according to the invention, said supports are designed to be used with very small amounts of control nucleic acid adsorbed thereon in a reproducible fashion. This certainly is useful in the case of PCR, especially real-time and quantitative PCR. Prior art supports were not designed for such small amounts of nucleic acids.

Said solid support according to the invention can have any shape, as long as it retains its essential features.
In a preferred embodiment, said solid support comprises at least one membrane. According to the invention, said membrane can advantageously be selected from the group of cellulose, cellulose-derived materials including chemically-treated celluloses, glass fibers, nylons, polyethersulfones, polypropylene, woven porous polymers, non-woven porous polymers, PTFE, porous glasses, and PVDF. Other suitable materials could possibly be used for said membrane, provided that they are absorbent materials, whose composition and structure allow for non-covalent adsorption of said control nucleic acid, and which is, or has been heat-treated and/or chemically treated so as to be, essentially devoid of any enzymatic activity. The skilled person would know such materials. In a preferred embodiment, said membrane material is selected from the group constituted by cellulose, cellulose-derived materials including chemically-treated celluloses, glass fibers, nylons, polyethersulfones, and polypropylene. More preferably, said membrane material is selected from the group of cellulose, cellulose derived materials and nylons. The preferred materials according to the invention are cellulose and nylons, among which cellulose is most preferred.
Said membrane can have various thicknesses, as can be judged by those skilled in the art, as long as it remains possibly easy to handle. Particularly preferred are membranes whose thickness makes it possible to be distributed with the help of a dispenser. In a preferred embodiment, said membrane has a thickness in the range 50-3000 microns, preferably 100-1500 microns, more preferably 150-1000 microns.

Any size and shape are possible for the solid support according to the present invention. In a preferred embodiment, said solid support is in the shape of a membrane. Most preferably, said membrane has the shape of a disk, a square, a rectangle or a strip. In another preferred embodiment, said membrane has the shape of a disk having a 6mm diameter, or a 6mm square. In yet another embodiment, said membrane has a surface in the range 10-500 mm², preferably 20-250 mm², more preferably 30-200 mm². Other sizes and shapes are also possible. The skilled person would know which sizes and shapes are suitable for said membrane. Advantageously according to the invention, said support will then fit into most, if not all, the usual containers and tubes known in molecular biology. For instance, said support will fit into a 1.5mL Eppendorf ® tube, as well as in most of the collection tubes for bodily fluids, e.g. haemolysis tubes.
Thus, the present invention relates to a solid support for at least one control nucleic acid, said solid support being specifically adapted for carrying out a method for the detection of at least one target nucleic acid according to the invention, said solid support comprising:
- at least one absorbent support made of a material whose composition and structure allow for non covalent adsorption of said control nucleic acid onto said solid support, and which is or has been heat-treated and/or chemically-treated so as to be essentially devoid of any enzymatic activity;
- at least one carrier agent adsorbed thereon, which facilitates the adsorption of said control nucleic acid onto said solid support and/or facilitates the desorption of said control nucleic acid from said solid support and/or promoting the stability of said control nucleic acid on said solid support, especially in the course of storage, substantially without affecting the primary sequence of said control nucleic acid;
- optionally, said control nucleic acid adsorbed thereon;
wherein said carrier agent is selected from the group constituted by:
- nucleic acids unrelated or heterologous to said control nucleic acid and/or to said target nucleic acid, so as to generally not interfere in the detection method;
- carrier agents of proteic nature.
Another object of the present invention is to provide with a series of supports which comprises a plurality of supports according to the invention, such that said series of supports advantageously provides with a calibration range of said control nucleic acid.
By plurality, it is meant at least three, preferably at least five or ten.
In a preferred embodiment, each of said supports from said series carries a different standardized amount of the same control nucleic acid adsorbed thereon, such that said series of supports provides with a calibration range of said control nucleic acid. Such ranges are particularly useful for the detection methods according to the present invention, and thus provide with calibration range which can be used especially when detection includes quantification. The skilled person would know how to choose said standardized amounts, depending upon the nature of said control nucleic acid and upon the detection carried out. In one embodiment according to the present invention, said series of solid supports provide with standardized amounts in the range of 10-10⁸ copies, preferably 10²-10⁶ copies, more preferably 10²-10⁵ copies. In another embodiment, said range is preferably 20-500 copies, more preferably 50-100 copies. Such ranges are particularly adapted to standard nucleic acid detection methods, including quantitative PCR.

It is a further object of the present invention to provide with a process for the manufacture of a solid support according to the invention.
In a preferred embodiment, at least part of, preferably the entirety of said process is performed under nuclease-free conditions.
According to the invention, there is provided a process for the manufacture of a solid support according to the invention, said process comprising the steps of:
- providing with an absorbent support material;
- heat-treating and/or chemically-treating said support, so as to essentially remove any nuclease activity;
- depositing at least one carrier agent onto said support material; and,
- optionally, depositing at least one control nucleic acid onto said support material so as to adsorb the desired amount of said control nucleic acid onto said support.
The skilled person would know which heat-treating and/or chemically-treating step(s) are suitable for essentially removing any nuclease activity. This usually involves physical and or chemical action to inhibit, inactivate, or degrade nucleases. In a preferred embodiment, said heat treatment is performed at a temperature greater than or equal to 100°C. For instance, said heat-treatment step can be performed from 100°C to 121°C or from 100°C to 190°C, preferably from 100°C to 180°C. In another preferred embodiment, said chemically-treating step can comprise at least one RNase inhibitor treatment, such as DEPC (Diethyl-pyrocarbonate) treatment.
Depositing steps are known to those skilled in the art. In a preferred embodiment, said carrier agent (resp. control nucleic acid) is provided in solution. Preferably, the concentration thereof is known. The solvent can be chosen among the suitable solvents therefore, as known by those skilled in the art. Example of such solvents include water, distilled water, nuclease-free water, TE, etc. (Molecular Cloning: A Laboratory Manual, Maniatis, Fritsch, and Sambrook, CSHL Press). In a most preferred embodiment, said depositing step(s) comprise(s) a spotting step. Said spotting step can be performed with any suitable spotting device known in the art, for instance, with a pipette, a micropipette, a multi-channel pipette, an automatic pipetting device, and the like, or a combination thereof. The skilled person is aware of the suitable devices for use in said spotting step. The volume to be spotted can be chosen by the skilled person, depending upon the desired amount of said carrier agent (resp. control nucleic acid) solution to be spotted, and the concentration of said solution. In a preferred embodiment, said depositing step comprises at least one step of drying. Said step can be achieved in several ways, as is known to the skilled person. In particular, in a preferred embodiment, the drying step is performed at a temperature greater than 40°C, preferably greater than 50°C, more preferably more than 60°C. In another preferred embodiment, the drying step is performed at a temperature in the range from 45°C to 70°C, preferably at around 60°C. Advantageously according to the invention, said adsorbed control nucleic acid is very stable, due to possible storage under dry conditions. This storage mode increases stability, and also allows for easy handling, as compared to liquid storage. In addition, according to the invention, said control nucleic acid can generally be stored for at least one year at room temperature, whereas liquid storage typically requires cooling means (fridge, freezer for instance) .

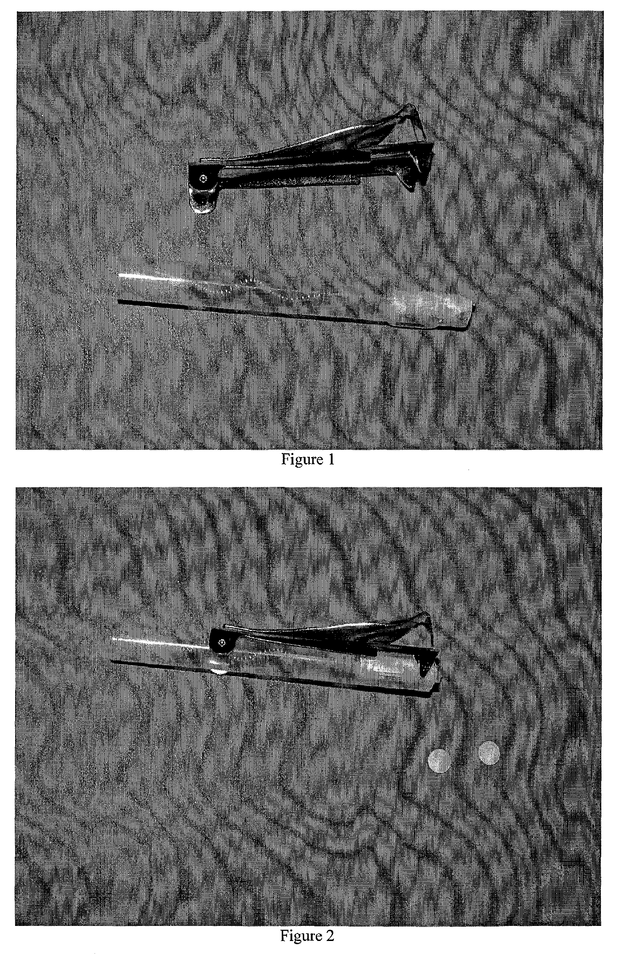

It is yet a further object of the present invention to provide with a kit.
According to the invention, there is provided a kit comprising:
- at least one solid support and/or at least one series of solid supports according to the invention, as described above;
- optionally, a dispenser for distributing said solid support into a container; and,
- instructions for the use thereof.
Advantageously according to the invention, said kit comprises instructions for the use thereof. Said instructions can advantageously be a leaflet, a card, or the like. Said instructions can also be present under two forms: a detailed one, gathering exhaustive information about the kit and the use thereof; and a quick-guide form or a memo, gathering the essential information needed for the use thereof.
According to the invention, said kit may comprise a dispenser for distributing said solid support into a container, e.g. tubes, especially PCR reaction tubes or PCR plates. A possible embodiment for said dispenser is shown on figures 1 and 2. Said dispenser allows for easy handling of said supports, without any direct contact to the manipulator, and advantageously decreases the risks of contaminations through manipulation or touching of said support. Contamination should be avoided as much as possible, especially in the case of PCR reactions. Said dispenser can advantageously be a semi-automatic dispenser for directly distributing said solid support into said container.
Said kit can serve various purposes. In some preferred embodiments, said kit is selected from the group constituted by:
- Kit for nucleic acid extraction and/or purification;
- Kit for nucleic acid detection;
- Kit for nucleic acid amplification, including PCR amplification, RT-PCR amplification, real-time PCR, quantitative PCR;
- Kit for the diagnosis of a disease or a condition.
Advantageously according to the invention, said kit may further comprise additional reagents useful for carrying out nucleic acid extraction and/or nucleic acid purification and/or nucleic acid further detection and/or nucleic acid amplification.
In a preferred embodiment, said kit according to the invention is a kit for PCR amplification. In that case, said kit may comprise a PCR reagent mix, containing at least some of the reagents required for the amplification reaction. In more preferred embodiment, said kit is designed for quantitative PCR, or as a diagnostics kit. Such a diagnostics kit is suitable for the diagnosis of a disease or a condition by detection of a nucleic acid target template in a sample, and/or by measurement of the amount of such a target template in said sample.

Hence, the present invention provides with methods, devices (solid supports), and kits for the detection of at least one target nucleic acid.
In this respect, the present application relates to many fields, like environment, agriculture, quality control, ... In particular, this invention relates to the field of diagnostics, and diagnostics methods. The abovementioned supports, processes, methods and kits can be used in the framework of a diagnostics procedure. For example, applications include detection and/or dosage of pathogen nucleic acid sequences, e.g. in a patient's biological sample.
By diagnostics, we understand any method contributing to the determination or assessment of a disease, a condition, or a state, pathological or not, irrespective of its stage.

The advantages of the products, processes and methods according to the invention will become apparent from the following examples, which are given below as mere illustrations, and are non limitative.

### Brief description of the drawings

**Figure 1** shows the two parts of a semi-automatic dispenser for the solid supports according to the invention.
**Figure 2** shows the same dispenser along with 2 solid supports according to the invention.

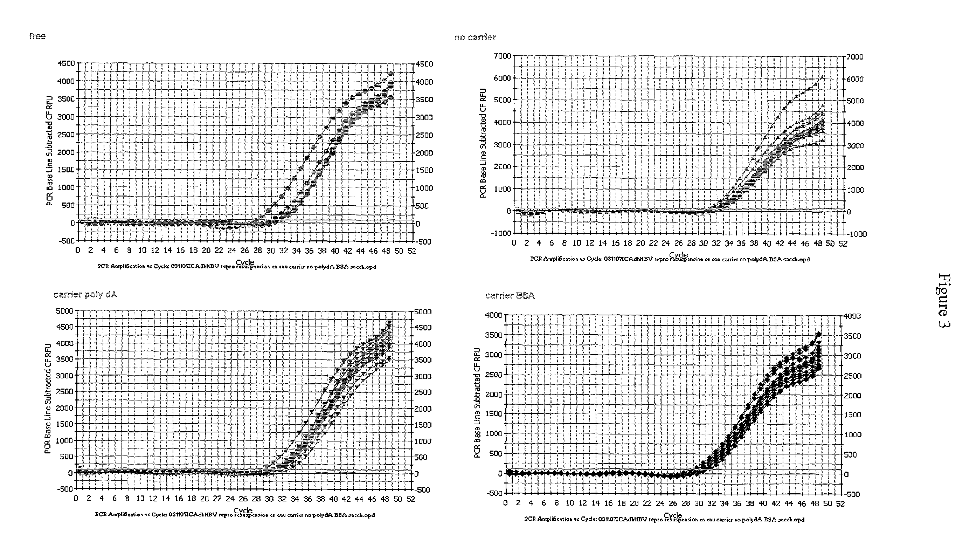

**Figure 3** shows the real-time PCR fluorescence curves, plotting the fluorescence signal (RFU, relative fluorescence units) versus the number of cycles, obtained under free conditions ("free"), or using a membrane without carrier ("no carrier"), or using a membrane according to the invention with either BSA ("carrier BSA") or polydA ("carrier polydA") as carrier agent adsorbed thereon, after resuspension in water (control nucleic acid is a DNA fragment).

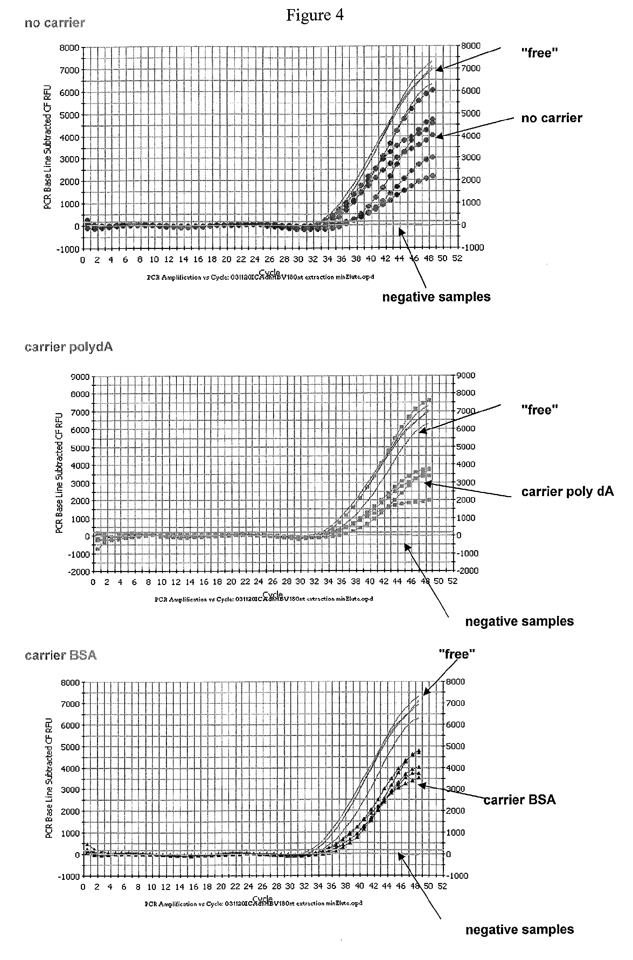

**Figure 4** shows the real-time PCR fluorescence curves, plotting the fluorescence signal (RFU, relative fluorescence units) versus the number of cycles, obtained using a membrane without carrier ("no carrier"), or using a membrane according to the invention with either BSA ("carrier BSA") or polydA ("carrier polydA") as carrier agent adsorbed thereon, after nucleic acid extraction (control nucleic acid is a DNA fragment).

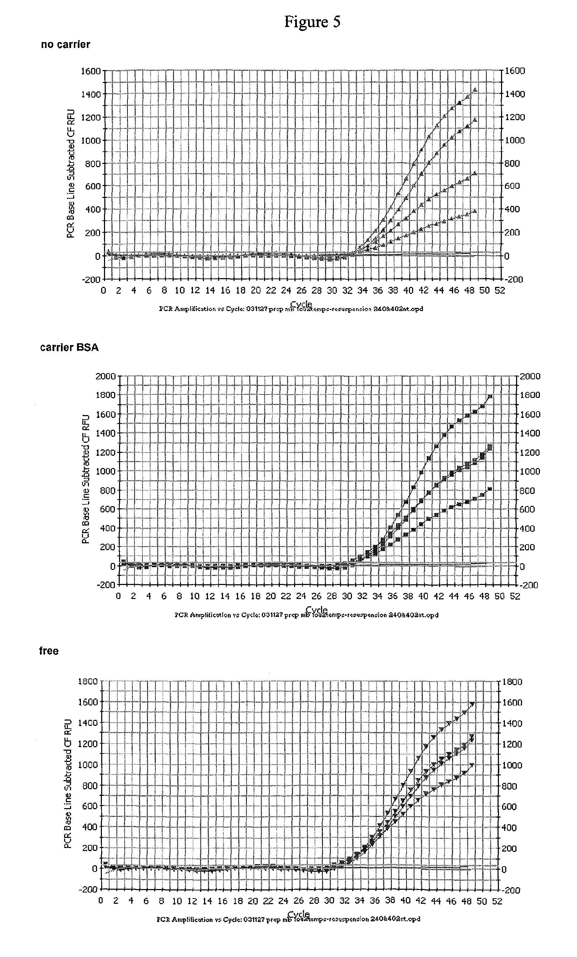

**Figure 5** shows the real-time RT-PCR fluorescence curves, plotting the fluorescence signal (RFU, relative fluorescence units) versus the number of cycles, obtained under free conditions ("free"), or using a membrane without carrier ("no carrier"), or using a membrane according to the invention with BSA as carrier agent adsorbed thereon ("carrier BSA"), after resuspension in water (control nucleic acid is an RNA fragment).

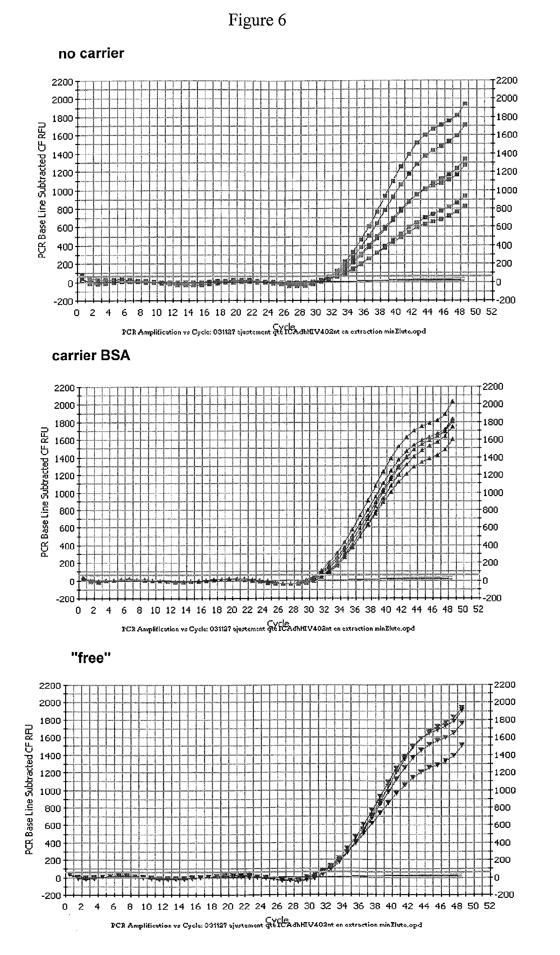

**Figure 6** shows the real-time RT-PCR fluorescence curves, plotting the fluorescence signal (RFU, relative fluorescence units) versus the number of cycles, obtained under free conditions ("free"), or using a membrane without carrier ("no carrier"), or using a membrane according to the invention with BSA as carrier agent adsorbed thereon ("carrier BSA"), after nucleic acid extraction (control nucleic acid is an RNA fragment).

### Examples

### Example 1: Preparation of a solid support according to the invention

Reagents:
- Membrane Whatman 920 µm (ref. whatman: 3017915 ; ref. VWR : A7199006) (Grade 17chr, smooth cellulose, 413g/m²)
- Water + 0.1% DEPC autoclaved
- Carrier agent:
   ■ BSA Invitrogen life biotechnologies (ref. 155561-020), or
   ■ polydA Amersham Biosciences (ref. 27-7836-01).

The membranes are cut into square pieces (6mmx6mm) with RNase-free tools (scissors, scalpel, pencil and ruler are cleaned with RNase Away ® ) under RNase-free conditions.

Under RNase free conditions (Clean bench and hood with RNase Away ® ):
- Wet the membranes with a solution of water + DEPC 0.1% (Diethyl pyrocarbonate) ('water + DEPC').
- Put the membranes in a closing box, and sterilize 30 minutes at 100°C until they are dried (this step also offers the advantage of inactivating the DEPC for subsequent use of the membranes); the membranes can now be stored for several days at room temperature;
- Dry 10 minutes at 60°C;
- Spot a solution containing the desired carrier agent, spot a suitable volume so as to adsorb the desired amount (for example 6µg polydA or 20µg BSA);
- polydA solutions can be stored as aliquots in water + DEPC at a suitable concentration;
- BSA is diluted extemporarily from a stock solution with water DEPC;
- Under the hood, spot a solution containing double stranded or single stranded DNA or RNA (as control nucleic acid): 5µL per membrane (advantageously, the number of copies spotted is controlled. For example, the number of copies can be in the range 10-10⁸ copies per 10µL) ;
- Dry 10 minutes at 60°C.
The membrane is now ready to use. Alternatively, the membrane can be kept overnight at 4°C, e.g. in an Eppendorf® tube.

### Example 2: comparison of support materials

Various membrane types were tested as solid supports.
The preferred groups of materials are summarized below:

**Table 1:**

| |
|---|
| Cellulose |
| Nylons |
| Polyethersulfone |
| Polypropylene |
| Cellulose acetate 0.2 µm |
| Isocode™ ID |
| Glass fibre |

Particularly good results were obtained for the following materials:
- Cellulose membrane, e.g. Whatman Grade 1, ref. 1001917
- Nylon membrane, e.g. Biorad Zeta Probe, ref. 1620153.

Advantageously, the membranes and supports according to the invention display the following characteristics:
- They are easy to handle, rather rigid, and are not subject to electrostatic effects.
- They allow for reproducible nucleic acid desorption.
- They perform a high release rate in water medium, and after extraction.
- Release is reproducible after resuspending the membrane in water and after extraction.
- They are substantially unaffected in the course of extraction, especially they are resistant to chemical treatments, e.g. ethanol and lysis buffer treatments. In particular, their structure and mechanical properties remains essentially unchanged after such treatments.
- They are heat resistant, in the sense that their structure and mechanical properties remain substantially unaltered after a heat treatment at 100°C or higher, especially for more than 15, 20, or 30 minutes.

### Example 3: Resuspension of control nucleic acids in water

- Membranes are prepared according to Example 1.
- Membranes are placed in Eppendorf ® tubes (1.5mL), one membrane per tube).
- Add 300 µL water + DEPC and close the tube.
- Turn the tube upside down once and vortex 15 seconds at full speed.
- Incubate for one hour at room temperature, making sure that the membrane is soaked in the water + DEPC. This step allows for the reproducible desorption of the control nucleic acid into the water + DEPC.
- Discard membrane using RNase Away® -treated tweezers.
- Clean the tweezers each time.
- Store the obtained samples (elutions) at -80°C.

### Example 4: Nucleic acid extraction using a solid support according to the invention

A solid support according to the invention can be used as an internal quantitative control, as detailed below:
Nucleic acid extraction (using a QIAamp™ MinElute™ Vacuum kit, Qiagen, ref. 57714):
   - Prepare a solid support (membrane) according to the invention, as in Example 1.
   - Starting material: 500 µl serum and/or plasma in a 2 ml tube.
   - Add 75 µl of protease (according to the manufacturer's instructions) and vortex briefly.
   - Add 500 µl of Lysis buffer (according to the manufacturer's instructions) and vortex briefly.
   - Add the membrane.
   - Vortex briefly.
   - Recover and purify the nucleic acids (according to the manufacturer's instructions)
   - Final elution in 60 µl.

The resulting elution can be used either in a PCR reaction, or in an RT-PCR reaction, as detailed in the following examples:

### Example 5: PCR amplification with an external control

- 25 µl of the elution as obtained in Example 3 are placed in a ready to use PCR mix (Platinium™ Quantitative PCR Super-Mix-UDG, Invitrogen, ref. 11730-017) with specific primers and a fluorescent probe.
- Mix gently with a tip.
- Run a real-time PCR.

Result interpretation: for each assay, one determines a threshold cycle (Ct) which is the level of fluorescence that is considered to be significantly above the background level of fluorescence measured in the early cycles of the amplification. The Ct value is inversely proportional to the concentration of control template. So the higher the Ct, the lower the concentration of control template.

The results of the PCR run obtained after resuspension of the membranes in water are presented in Table 2 (table with Ct values and maximal fluorescence value for each curve) and in figure 3 (representation of curves).

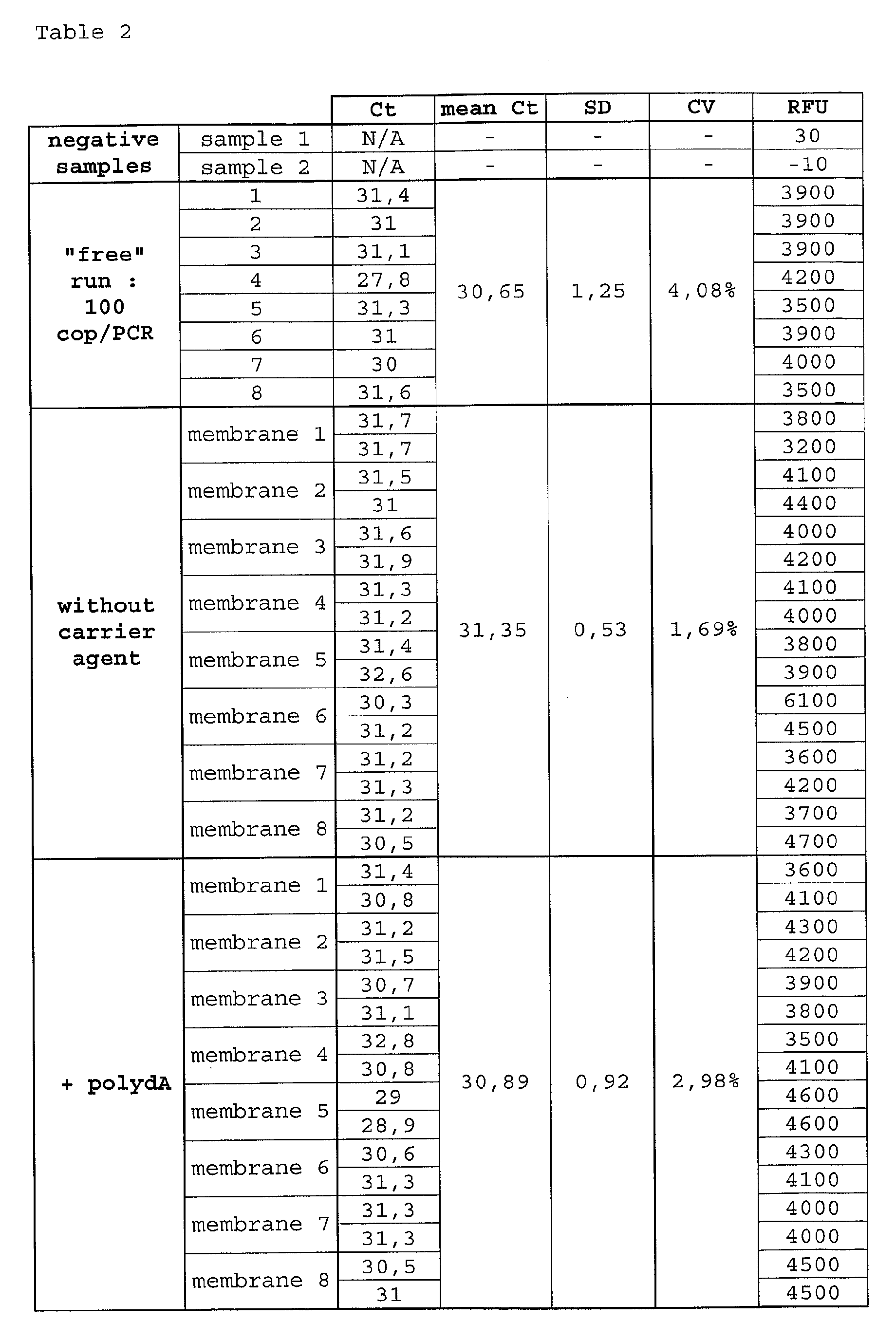

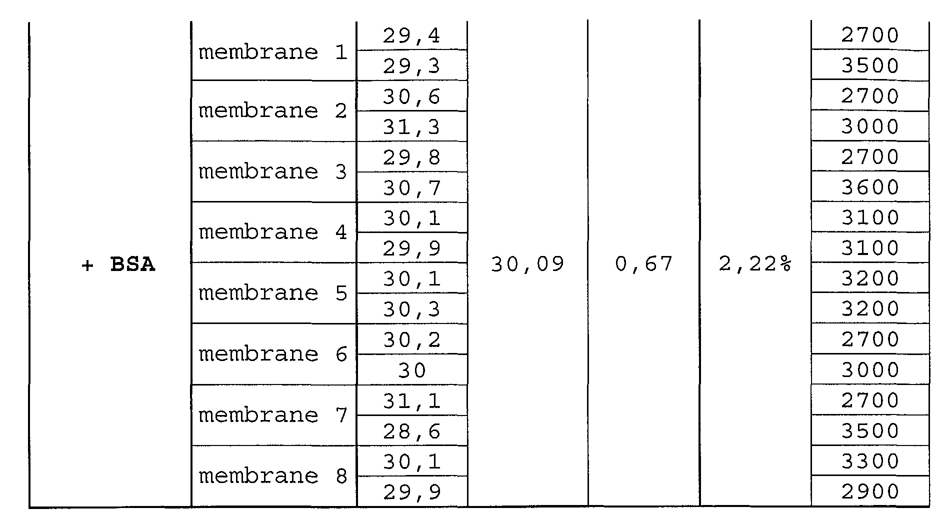

### Table 2

Results of real time PCR using a membrane (with or without polydA or BSA as carrier agent adsorbed thereon) spotted with 1200 copies of DNA amplified fragment per membrane (control template) and resuspended in 300 µl water

Membranes with or without polydA or BSA as a carrier agent adsorbed thereon were spotted with 1200 copies of the control nucleic acid (here a DNA fragment) per membrane as described in Example 1, and resuspended in 300 µl of water as described in Example 3. 25 µl of this resuspension/elution is then used in a PCR amplification reaction and compared to the direct addition in the PCR mix of 25 µl containing 100 copies of the same control nucleic acid ("free" run, i.e. free conditions, without the solid support according to the invention, i.e. under "free" conditions, the control nucleic acid is supplied from a solution and not from a solid support). For free conditions, 8 PCR replicates have been tested. For membranes with or without a carrier agent adsorbed thereon, 8 membranes have been tested with a PCR duplicate per membrane.

Conclusions:
- The amount of control nucleic acid spotted on the membrane has been calibrated in order to get a Ct value close to the Ct value obtained in free conditions.
- The mean Ct obtained with a membrane without any carrier agent is close to the mean Ct obtained in free conditions with a better reproducibility when using a membrane according to the invention.
- The mean Ct obtained on a membrane with carrier is slightly lower that the mean Ct obtained with a membrane without any carrier agent, corresponding to a better release of the control nucleic acid when a carrier agent is used.
- The solid support according to the invention can be used for external controls, qualitative controls, quantitative controls, external quantitative controls in a PCR amplification reaction.

### Example 6: PCR amplification with an internal control

- 25 µl of the elution as obtained in Example 4 are placed in a ready to use PCR mix (Platinium™ Quantitative PCR Super-Mix-UDG, Invitrogen, ref. 11730-017) with specific primers and a fluorescent probe.
- Mix gently with a tip.
- Run a real-time PCR.

Result interpretation: for each assay, one determines a threshold cycle (Ct) which is the level of fluorescence that is considered to be significantly above the background level of fluorescence measured in the early cycles of the amplification. The Ct value is inversely proportional to the concentration of control template. So the higher the Ct, the lower the concentration of control template.

The results of the PCR run obtained after nucleic acid extraction using the membranes according to the invention as described in Example 4 are presented in Table 3 (table with Ct values and maximal fluorescence value for each curve) and in figure 4 (representation of curves).

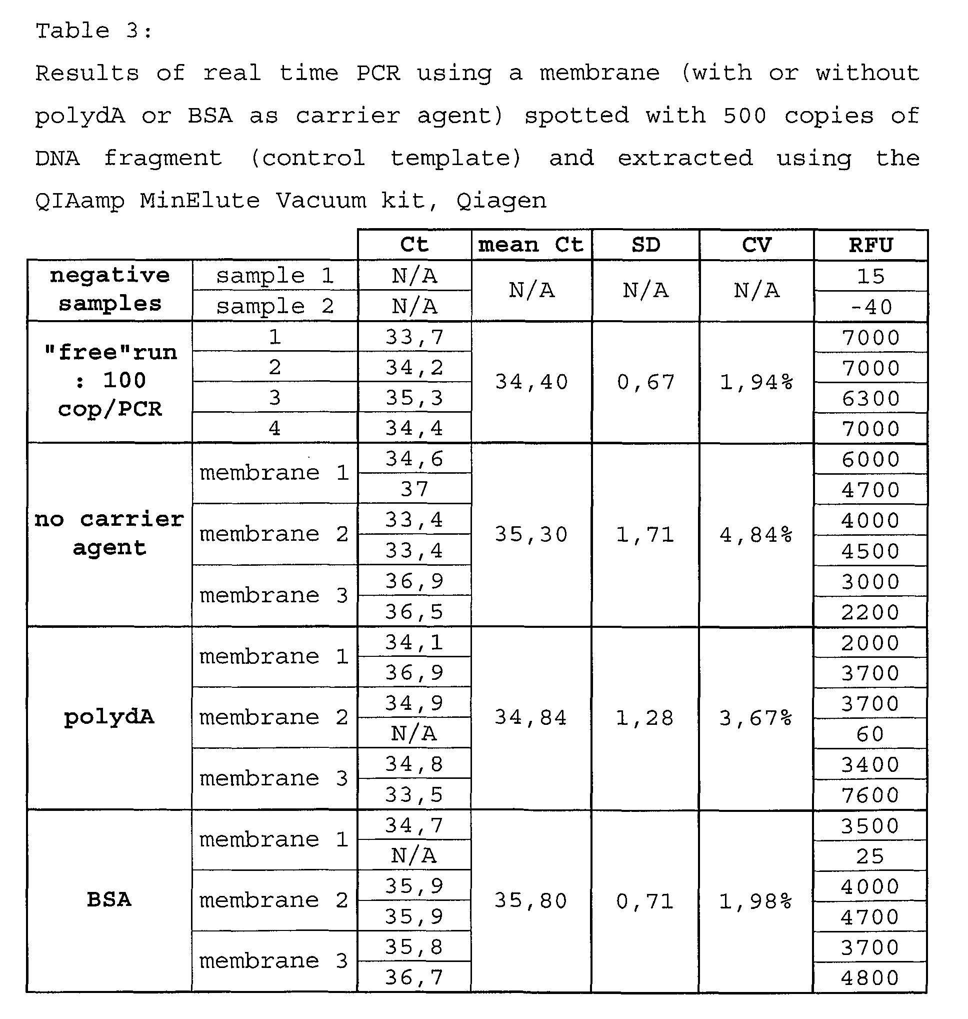

Membranes with or without polydA or BSA as a carrier agent were spotted with 500 copies of control nucleic acid (here a DNA fragment) per membrane as described in Example 1. After nucleic acid extraction as described in Example 4, 25 µl of the elution volume (final volume = 60 µl) is then used in a PCR amplification reaction and compared to the direct addition in the PCR mix of 100 copies of the same control nucleic acid ("free" run, free conditions, without the solid support according to the invention, i.e. from a solution). For free conditions, 4 PCR replicates have been tested. For membranes with or without a carrier agent adsorbed thereon, 3 membranes have been tested with a PCR duplicate per membrane.

Conclusions:
- The amount of control nucleic acid spotted on membrane has been calibrated in order to get a Ct value close to the Ct value obtained in free conditions.
- The better Ct value is obtained with a membrane having a polydA carrier agent adsorbed thereon, but the better Ct reproducibility is obtained with the membranes having a BSA carrier agent adsorbed thereon.
- As observed on figure 4, the maximal fluorescence values obtained with BSA as the carrier agent are the most homogeneous.

It is thus possible, according to the invention, to detect and quantify by PCR the amplification of a target template from the serum/plasma sample, in relation to the amplification of the control. The control thus serves for the two steps, extraction and amplification.

### Example 7: RT-PCR amplification with an external control

- 10 µl of the elution as obtained in Example 3 are placed in a ready to use RT-PCR mix (QuantiTect™ Probe RT-PCR kit, Qiagen, ref. 204443) with specific primers and a fluorescent probe.
- Mix gently with tip.
- Run a real-time RT-PCR.

Result interpretation: for each assay, one determines a threshold cycle (Ct) which is the level of fluorescence that is considered to be significantly above the background level of fluorescence measured in the early cycles of the amplification. The Ct value is inversely proportional to the concentration of control template. So the higher the Ct, the lower the concentration of control template.

The results of the RT-PCR run obtained after resuspension of the membranes according to the invention in water are presented in Table 4 (table with Ct values and maximal fluorescence value for each curve) and in figure 5 (representation of curves).

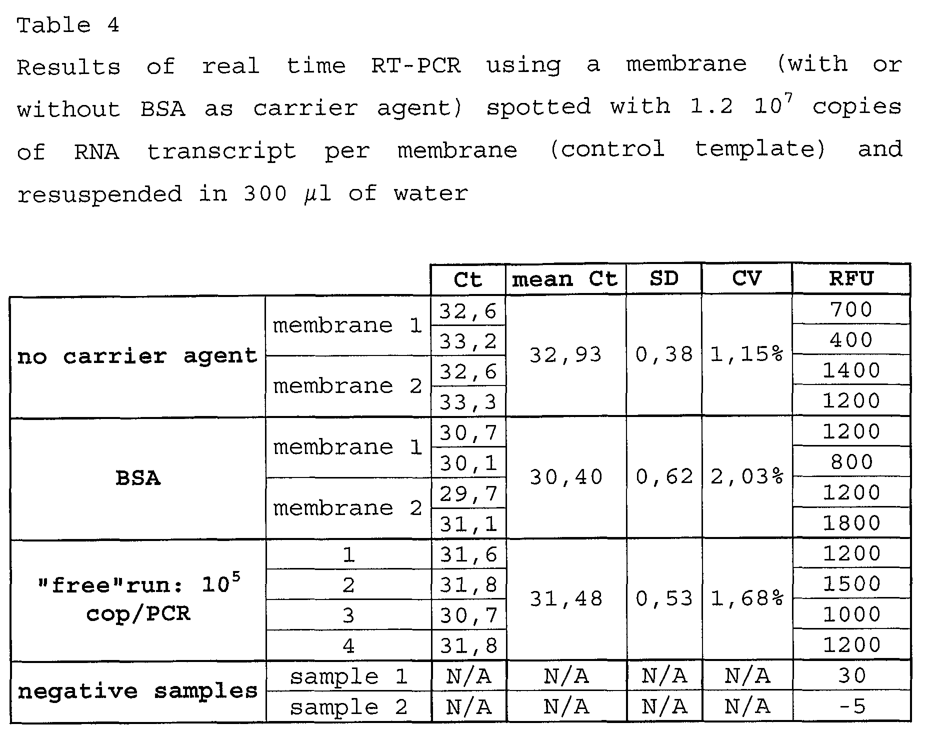

Membranes with or without carrier BSA were spotted with 1.2x10⁷ copies of an RNA transcript as control nucleic acid per membrane as described in Example 1, and resuspended in 300 µl of water as described in Example 3. 10 µl of this resuspension is then used for an RT-PCR and compared to the direct addition in the RT-PCR mix of 10⁵ copies of the same control nucleic acid (from a solution, "free" run). For free conditions, 4 replicates have been tested. For membrane with or without a carrier agent, 2 membranes have been tested with a duplicate PCR per membrane.

Conclusions:
- The amount of RNA transcript (control nucleic acid) spotted on the membrane has been calibrated in order to get a Ct value close to the Ct value obtained in free conditions.
- The mean Ct obtained on membranes with or without a carrier agent are close to the mean Ct obtained in free conditions with a better release of the control nucleic acid obtained when BSA is used as a carrier agent.

### Example 8: RT- PCR amplification with an internal control

- 10 µl of the elution as obtained in Example 3 are placed in a ready to use RT-PCR mix (QuantiTect™ Probe RT-PCR kit, Qiagen, ref. 204443) with specific primers and a fluorescent probe.
- Mix gently with tip.
- Run a real-time RT-PCR.

Result interpretation: for each assay, one determines a threshold cycle (Ct) which is the level of fluorescence that is considered to be significantly above the background level of fluorescence measured in the early cycles of the amplification. The Ct value is inversely proportional to the concentration of control template. So the higher the Ct, the lower the concentration of control template.

The results of the run obtained after nucleic acid extraction using the membranes are presented in Table 5 (table with Ct values and maximal fluorescence value for each curve) and in figure 6 (representation of curves).

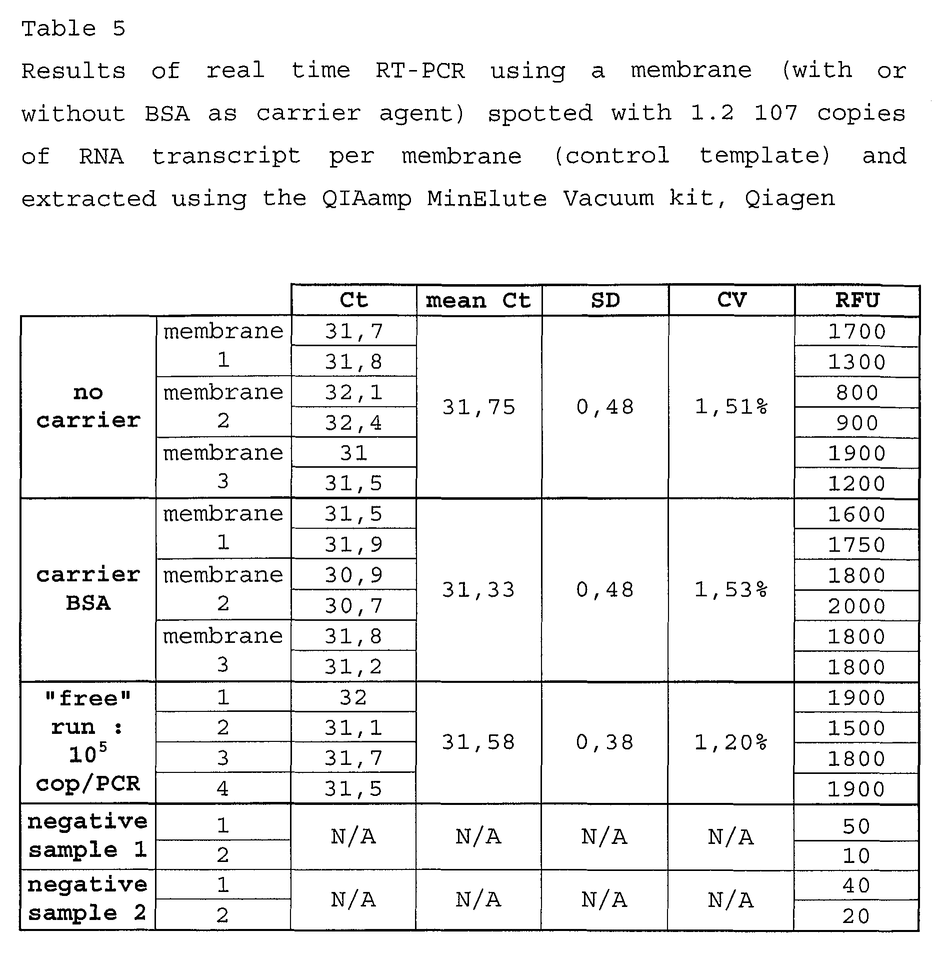

Membranes with or without carrier BSA were spotted with 1.2x10⁷ copies of RNA transcript (control nucleic acid) per membrane as described in Example 1. After nucleic acid extraction as described in Example 4, 10 µl of the elution volume (final volume = 60 µl) were then used for RT-PCR and compared to the direct addition in the RT-PCR mix of 10⁵ copies of the same control nucleic acid (from a solution, "free" run). For free conditions, 4 replicates have been tested. For membranes with or without a carrier agent, 3 membranes have been tested with 2 duplicates RT-PCR per membrane.

Conclusion :
- The amount of RNA transcript (control nucleic acid) spotted on membrane has been calibrated in order to get a Ct value close to the Ct value obtained in free conditions
- The mean Ct obtained with a membrane with or without BSA as a carrier agent are close to the mean Ct obtained in free conditions.
- As observed on the curves, the maximal fluorescence values obtained with BSA as carrier agent are the most homogeneous.

It is thus possible, according to the invention, to detect and quantify by RT-PCR the amplification of a target template from the serum/plasma sample, in relation to the amplification of the control. The control thus serves for the two steps, extraction and amplification.

The skilled person can appreciate that the present invention can incorporate any number of the preferred features described above.
All citations mentioned herein are hereby incorporated by reference in their entirety.
Other embodiments of the present invention are not presented here, which are obvious to those skilled in the art, and thus are within the scope and the spirit of the present invention.

## Claims

1. Method to determine the presence or the absence of at least one target nucleic acid by reference to at least one control nucleic acid, which comprises processing said target nucleic acid so as to allow its detection, submitting said control nucleic acid to comparable processing conditions, and validating or invalidating the detection result obtained for said target nucleic acid by comparing it to the detection result obtained for said control nucleic acid,
wherein said control nucleic acid is provided by a solid support onto which it is adsorbed, and from which a definite amount thereof is to be desorbed, whereby there is provided an essentially quantitatively reproducible and controlled amount of said control nucleic acid for submission to said comparable processing conditions.

2. Method according to claim 1,
said method comprising the steps of:
- providing with said solid support comprising said at least one control nucleic acid adsorbed thereon, and contacting said solid support with a liquid medium, so as to allow a definite amount of said control nucleic acid to be desorbed from said solid support into said liquid medium, substantially without affecting the primary sequence of said control nucleic acid;
- optionally, further processing the resulting liquid medium containing said control nucleic acid;
- submitting said control nucleic acid which is contained in or originating from said resulting, optionally further processed, liquid medium, to comparable processing conditions, so that it constitutes a processing control by reference to the processing of said target nucleic acid;
- determining whether said target nucleic acid is detected or not, and determining whether said control nucleic acid is detected or not;
- comparing the target nucleic acid detection result to the one of said control nucleic acid, so as to validate or invalidate the target nucleic acid detection result;
wherein said processing comprises at least one processing step selected from the group constituted by amplification, including PCR amplification, gel electrophoresis, Southern analysis, northern analysis, hybridization including probe-mediated hybridization, and combinations thereof.

3. Method according to any one of claims 1-2, wherein said solid support comprises at least one membrane.

4. Method according to the preceding claim, wherein said membrane material is selected from the group constituted by cellulose, cellulose-derived materials including chemically-treated celluloses, glass fibers, nylons, polyethersulfones, polypropylene, woven porous polymers, non-woven porous polymers, PTFE, porous glasses, and PVDF.

5. Method according to any one of claims 3-4, wherein said membrane material is selected from the group constituted by cellulose, cellulose-derived materials including chemically-treated celluloses, glass fibers, nylons, polyethersulfones, and polypropylene; preferably from the group constituted by cellulose, cellulose-derived materials and nylons.

6. Method according to any one of claims 3-5, wherein said membrane has a thickness in the range 50-3000 microns, preferably 100-1500 microns, more preferably 150-1000 microns.

7. Method according to any one of claims 3-6, wherein said membrane has the shape of a disk, a square, a rectangle or a strip.

8. Method according to any one of claims 1-7, wherein said solid support further comprises at least one carrier agent adsorbed thereon.

9. Method according to the preceding claim, wherein said carrier agent is selected from the group constituted by:
- nucleic acids unrelated or heterologous to said control nucleic acid, so as to substantially not interfere in the detection processing of said control nucleic acid;
- proteins.

10. Method according to any one of claims 8-9, wherein said carrier agent is a nucleic acid unrelated or heterologous to said control nucleic acid and also unrelated or heterologous to said target nucleic acid, so as to substantially not interfere in the detection processing of said control and said target nucleic acids.

11. Method according to any one of claims 8-10, wherein said carrier agent is selected from the group constituted by polyA, polyG, polyC, polyT, polydA, polydG, polydC, polydT, polydU, homopolydeoxyribonucleotides, homopolyribonucleotides , block-polymers of deoxyribonucleotides, block-polymers of ribonucleotides, block-polymers of deoxyribonucleotides and ribonucleotides, and mixtures thereof.

12. Method according to any one of claims 8-10, wherein said carrier agent is selected from the group constituted by:
- fish DNA, such as herring DNA, especially herring sperm DNA, and salmon DNA, especially salmon sperm DNA, and mixtures thereof;
- albumins, especially bovine serum albumin (BSA).

13. Method according to any one of claims 8-11, wherein said carrier agent is selected from the group constituted by:
- homopolydeoxyribonucleotides, block-polymers of deoxyribonucleotides, and mixtures thereof;
- albumins, especially bovine serum albumin (BSA).

14. Method according to any one of claims 1-13, wherein said control nucleic acid is selected from the group constituted by positive controls, negative controls, internal controls, external controls, qualitative controls, semi-quantitative controls, quantitative controls, real-time amplification controls, and combinations thereof.

15. Method according to any one of claims 1-14, wherein said nucleic acid processing for detection comprises at least one nucleic acid extraction and/or purification step, prior to said nucleic acid detection step.

16. Method according to any one of claims 1-15, wherein said processing comprises a detection step involving at least one hybridization step.

17. Method according to any one of claims 1-16, wherein said processing comprises a detection step involving at least one PCR or RT-PCR, especially real-time and quantitative PCR or RT-PCR.

18. Method according to any one of claims 1-17, wherein said target nucleic acid detection is a quantitative detection, preferably a real-time quantitative detection.

19. Method according to any one of claims 1-18, wherein said control nucleic acid detection is a quantitative detection, preferably a real-time quantitative detection.

20. Solid support for at least one control nucleic acid, said solid support being specifically adapted for carrying out a method for the determination of presence or absence of at least one target nucleic acid by reference to at least one control nucleic acid according to any one of claims 1-19,
said solid support comprising:
- at least one absorbent support made of a material whose composition and structure allow for non covalent adsorption of said control nucleic acid onto said solid support, and which is or has been heat-treated and/or chemically-treated so as to be essentially devoid of any enzymatic activity;
- at least one carrier agent adsorbed thereon, which facilitates the adsorption of said control nucleic acid onto said solid support and/or facilitates the desorption of said control nucleic acid from said solid support and/or promoting the stability of said control nucleic acid on said solid support, especially in the course of storage, substantially without affecting the primary sequence of said control nucleic acid;
- optionally, said control nucleic acid adsorbed thereon;
wherein said carrier agent is selected from the group constituted by:
- nucleic acids unrelated or heterologous to said control nucleic acid and/or to said target nucleic acid, so as to generally not interfere in the detection method;
- carrier agents of proteic nature.

21. Solid support according to the preceding claim, wherein said carrier agent is a nucleic acid unrelated to any naturally occurring human nucleic acid.

22. Solid support according any one of claims 20-21, wherein said carrier agent is a nucleic acid unrelated to any nucleic acid originating from nucleic acid originating from any naturally occurring agent being pathogen to a mammalian, especially to human.

23. Solid support according to any one of claims 20-22, wherein said carrier agent is selected from the group constituted by:
- polyA, polyG, polyC, polyT, polydA, polydG, polydC, polydT, polydU, homopolydeoxyribonucleotides, homopolyribonucleotides , block-polymers of deoxyribonucleotides, block-polymers of ribonucleotides, block-polymers of deoxyribonucleotides and ribonucleotides, and mixtures thereof;
- fish DNA;
- proteins, such as albumin.

24. Solid support according to any one of claims 20-22, wherein said carrier agent is selected from the group constituted by:
- homopolydeoxyribonucleotides such as polydA, block-polymers of deoxyribonucleotides, and mixtures thereof;
- herring DNA, especially herring sperm DNA, and salmon DNA, especially salmon sperm DNA, and mixtures thereof;
- albumins, such as bovine serum albumin (BSA).

25. Solid support according to any one of claims 20-24, wherein said carrier agent is selected from the group constituted by:
- homopolydeoxyribonucleotides such as polydA, block-polymers of deoxyribonucleotides, and mixtures thereof;
- albumins, such as bovine serum albumin (BSA).

26. Solid support according to any one of claims 20-25, wherein said carrier agent comprises :
- 0.1-50 µg of nucleic acids, preferably 1-10 µg, more preferably 4-8 µg, even more preferably 5-6 µg, or
- 2-100 µg of proteins (e.g. BSA), preferably 5-50 µg, more preferably 10-30 µg, even more preferably 15-20 µg.

27. Solid support according to any one of claims 20-26, wherein said support comprises at least one membrane.

28. Solid support according to the preceding claim, wherein said membrane material is selected from the group of cellulose, cellulose-derived materials including chemically-treated celluloses, glass fibers, nylons, polyethersulfones, polypropylene, woven porous polymers, non-woven porous polymers, PTFE, porous glasses, and PVDF.

29. Solid support according to any one of claims 27-28, wherein said membrane material is selected from the group constituted by cellulose, cellulose-derived materials including chemically-treated celluloses, glass fibers, nylons, polyethersulfones, and polypropylene.

30. Solid support according to any one of claims 27-29, wherein said membrane material is selected from the group of cellulose, cellulose derived materials and nylons.

31. Solid support according to any one of claims 27-30, wherein said membrane has a thickness in the range 50-3000 microns, preferably 100-1500 microns, more preferably 150-1000 microns.

32. Solid support according to any one of claims 27-31, wherein said membrane has the shape of a disk, a square, a rectangle or a strip.

33. Solid support according to any one of claims 27-32, wherein said membrane has a surface in the range 10-500 mm², preferably 20-250 mm², more preferably 30-200 mm².

34. Solid support according to any one of claims 20-33, wherein said enzymatic activity comprises nuclease activity, including DNAse and/or RNase activity.

35. Solid support according to any one of claims 20-34, wherein said solid support further comprises at least one control nucleic acid adsorbed thereon.

36. Solid support according to the preceding claim, wherein said control nucleic acid is selected from the group constituted by positive controls, negative controls, internal controls, external controls, qualitative controls, semi-quantitative controls, quantitative controls, real-time amplification controls, and combinations thereof.

37. Solid support according to any one of claims 35-36, wherein said control nucleic acid is adsorbed in an amount in the range 10-10⁸ copies, preferably 10²-10⁵ copies.

38. Solid support according to any one of claims 35-37, wherein said control nucleic acid is adsorbed in an amount in the range 10-1000 copies, preferably 20-500 copies, more preferably 50-100 copies.

39. Series of supports which comprises a plurality of supports according to any one of claims 35-38, wherein each of said supports carries a different standardized amount of the same control nucleic acid adsorbed thereon, such that said series of supports provides with a calibration range of said control nucleic acid, preferably in the range 10-10⁸ copies, more preferably 10²-10⁵ copies, even more preferably 20-500 copies, most preferably 50-100 copies.

40. Process for the manufacture of a solid support according to any one of claims 20-38, said process comprising the steps of:
- providing with an absorbent support material;
- heat-treating and/or chemically-treating said support, so as to essentially remove any nuclease activity;
- depositing at least one carrier agent onto said support material; and,
- optionally, depositing at least one control nucleic acid onto said support material so as to adsorb the desired amount of said control nucleic acid onto said support.

41. Process according to the preceding claim, wherein said heat treatment is performed at a temperature in the range from 100°C to 180°C.

42. Process according to any one of claims 40-41, wherein said chemically treating comprises a DEPC (Diethyl-pyrocarbonate) treatment.

43. Process according to any one of claims 40-42, wherein said depositing step(s) comprise(s) at least one spotting step.

44. Process according to any one of claims 40-43, wherein said depositing step(s) comprise(s) at least one drying step.

45. Process according to the preceding claim, wherein said drying step is performed at a temperature in the range from 45°C to 70°C, preferably at around 60°C.

46. Kit comprising:
- at least one solid support according to 20-38 and/or at least one series of solid supports according to claim 39;
- optionally, a dispenser for distributing said solid support into a container; and,
- instructions for the use thereof.

47. Kit according to the preceding claim, wherein said kit is selected from the group constituted by:
- Kit for nucleic acid extraction and/or purification;
- Kit for nucleic acid detection;
- Kit for nucleic acid amplification, including PCR amplification, RT-PCR amplification, real-time PCR, quantitative PCR;
- Kit for the diagnosis of a disease or a condition.
